# EUROPEAN PATENT APPLICATION

(11) **EP 4 825 400 A1**
(43) Date of publication of application: **21.10.2026**
(21) Application number: 26173165.7
(22) Date of filing: 17.04.2026
(51) Int. Cl.: A61L 27/52, A61L 27/02, A61L 27/20

(54) **A BIOGEL COMPOSITION FOR MULTITISSUE REGENERATION**

(30) Priority: 17.04.2025 PT 2025120223
(71) Applicant: ANDRADE OLIVEIRA DE SOUSA, JORGE MIGUEL, 3050-330 Mealhada (PT)
(72) Inventor: ANDRADE OLIVEIRA DE SOUSA, JORGE MIGUEL, 3050-330 Mealhada (PT)
(74) Representative: Pereira da Cruz, Joao

(57) **Abstract**

The present disclosure relates to a biogel composition for multitissue regeneration (1), comprising at least one gelling agent (10) configured to form or contribute to the formation of a three-dimensional polymeric network (100), and at least one ionic-modifying agent (20) comprising ferrous ions (Fe²⁺) and/or ferric ions (Fe³⁺), configured to trigger, promote and/or modulate interactions (101) within the biogel composition (1), thereby leading to the formation and/or stabilization of the three-dimensional polymeric network (100), the said three-dimensional polymeric network (100) having viscoelastic behavior. The present disclosure further relates to a system for administering the biogel composition (1), a method for producing a biogel composition (1), and a method for administering the biogel composition (1) onto or within at least one biological surface.

## Description

### FIELD OF THE DISCLOSURE

The present disclosure relates to the field of biomaterials engineering, particularly medical devices intended for the protection, regeneration, support and/or temporary or permanent tissue replacement. It is applicable to several clinical areas, including dermatology, orthopedics, plastic surgery, dentistry and/or ophthalmology.

### PRIOR ART

Currently, wound healing and tissue regeneration rely on a wide range of materials with distinct physicochemical and biological mechanisms, including conventional gauzes, polymeric dressings and films, surgical sutures, collagen membranes, synthetic dermal substitutes, Platelet-Rich Fibrin (PRF), animal-derived biological materials, and biomaterials used in bone regeneration.

Conventional gauzes provide superficial wound coverage and absorb biological fluids, including exudates, acting primarily as physical barriers. However, they do not contribute to effective hemorrhage control or tissue regeneration. Polymeric dressings and films regulate wound moisture and serve as mechanical barriers, but may dry the wound or adhere to healing tissue, making removal difficult and potentially causing additional trauma. Surgical sutures approximate tissues but lack regenerative properties and do not ensure continuous superficial protection throughout the healing process.

Collagen membranes and other synthetic dermal substitutes function as tissue barriers and structural supports and are used in burns or dermal defects resulting from trauma, surgery, or pathology. Nevertheless, they are unsuitable for rapid application in physiological moist environments, including hemorrhagic conditions, and do not actively promote regeneration. They also frequently require fixation techniques and are associated with high costs.

PRF, an autologous material obtained from the patient's blood, provides growth factors relevant to regeneration and contributes to hemorrhage control through fibrin-matrix formation. However, its use depends on specific protocols and specialized equipment, such as centrifugation, and its properties vary with patient characteristics, making it a more invasive and technically demanding procedure. Moreover, PRF does not act as a stable mechanical barrier and is easily displaced in physiological moist environments.

Animal-derived biological materials and biomaterials used in bone regeneration, such as hydroxyapatite and calcium phosphates, are primarily applied in mineralized tissues, limiting their use to these contexts. They are not fully resorbable - i.e. capable of being degraded and absorbed by the body -, lack osteoinductive activity, and often require additional barrier membranes to prevent soft-tissue migration, while offering no contribution to hemorrhage control or soft-tissue regeneration.

Despite the variety of available solutions, none simultaneously provides barrier function, hemorrhage control, and promotion of tissue regeneration. As a result, multiple materials and procedures are often combined to achieve complementary effects, increasing procedural complexity and reducing effectiveness under actual physiological conditions, particularly in physiological moist environments such as those involving blood.

The present disclosure addresses these limitations through a biogel composition exhibiting viscoelasticity, adhesiveness, and absorption capacity suitable for application in physiological moist environments. This biogel composition promotes hemostasis and supports key biological processes, including wound healing and tissue regeneration. It may also be tailored for applications requiring modulation of tissue mechanical or rheological properties, such as viscosupplementation, and for the controlled release of bioactive agents, including pharmacological compounds.

### SUMMARY OF THE DISCLOSURE

The present disclosure relates to a biogel composition for multitissue regeneration, characterized by comprising:
- at least one gelling agent configured to form or contribute to the formation of a three-dimensional polymeric network;
- at least one ionic-modifying agent comprising ferrous ions (Fe²⁺) and/or ferric ions (Fe³⁺), and being configured to trigger, promote and/or modulate interactions within the biogel composition, thereby leading to the formation and/or stabilization of the said three-dimensional polymeric network;
the three-dimensional polymeric network having viscoelastic behaviour, resulting, at least in part, from interactions within the biogel composition.

The interactions triggered, promoted and/or modulated by the at least one ionic-modifying agent within the biogel composition may comprise at least ionic interactions.

The at least one ionic-modifying agent may comprise at least one iron salt selected from ferrous sulfate (FeSO₄), ferric sulfate (Fe₂(SO₄)₃), or any combination thereof.

The biogel composition may further comprise at least one hydration-modulating agent configured to bind and/or retain water and to contribute to at least one rheological property of the biogel composition; the at least one rheological property comprising at least one mechanical parameter selected from viscosity, elasticity, plasticity, shear-thinning behavior, and/or post-shear structural recovery.

The at least one hydration-modulating agent may comprise at least one polysaccharide, synthetic polymer, protein, or combination thereof; preferably a polysaccharide.

The polysaccharide may be hyaluronic acid; the hyaluronic acid having a molecular weight between 300000 Da and 2000000 Da, preferably between 500000 Da and 2000000 Da.

The biogel composition may further comprise at least one crosslinking agent configured to reinforce and stabilize the three-dimensional polymeric network; the at least one crosslinking agent being selected from genipin, 1,4-Butanediol Diglycidyl Ether (BDDE), carbodiimides, tannic acid, or any combination thereof.

The biogel composition may further comprise at least one thickening agent configured to contribute to the modulation and/or stabilization of the biogel composition, including in a physiological moist environment; the at least one thickening agent being selected from carboxymethylcellulose, carbomer, polyvinylpyrrolidone, poloxamers, hydroxyethyl cellulose, hydroxypropyl cellulose, gelatin, agarose, or any combination thereof.

The physiological moist environment may comprise the presence of at least one biological fluid selected from blood, saliva, interstitial fluid, exudate, or any combination thereof.

The biogel composition may further comprise at least one antioxidant agent configured to limit the oxidation of the at least one ionic-modifying agent; the at least one antioxidant agent being selected from ascorbic acid, sodium ascorbate, glutathione, N-acetylcysteine, or any combination thereof.

The biogel composition may be configured to provide controlled release of at least one bioactive agent; the controlled release of the at least one bioactive agent comprising the gradual diffusion of the said bioactive agent through the three-dimensional polymeric network and/or through controlled degradation of that three-dimensional polymeric network.

The at least one bioactive agent may be selected from an antimicrobial agent, an anti-inflammatory agent, a tissue-regeneration-promoting agent; a protein; a peptide; a nanostructured agent; a liposomal system; and/or any combination thereof; and the controlled release of at least one antimicrobial agent may be configured to confer antimicrobial activity, including antibacterial and/or antifungal activity, to the biogel composition.

The biogel composition may be configured as a mucoadhesive gel, foam, or in a liquid or semisolid form, and/or be configured to form, upon administration onto or within at least one biological surface, at least one continuous adherent layer.

In addition, the biogel composition may be configured to present, on or within at least one biological surface, a controlled absorption time greater than that of the at least one gelling agent in the absence of the at least one ionic-modifying agent.

The present disclosure further relates to a system for administering a biogel composition according to the present disclosure, comprising at least one biogel composition and at least one device configured to prepare, handle, and/or administer the biogel composition.

The present disclosure also relates to the uses of the biogel composition according to the present disclosure, namely as:
- a filling, coating, sealing, protective, stabilizing and/or regeneration material of at least one biological surface;
- a substitute for or complement to at least one filling material selected from collagen membranes, bone substitutes, connective-tissue substitutes, autologous grafts, and/or at least one suture material;
- a hemostatic agent and/or for promoting healing and/or regeneration of at least one biological surface.

Additionally, the present disclosure discloses the use of the biogel composition in three-dimensional bioprinting via digital modelling, preferably through Computer-Aided Design (CAD) and/or Computer-Aided Manufacturing (CAM).

Additionally, the present disclosure relates to a method for producing a biogel composition as described herein, comprising the steps of:
a) providing at least one gelling agent, preferably in an aqueous phase;
b) providing at least one ionic-modifying agent comprising ferrous ions (Fe²⁺) and/or ferric ions (Fe³⁺);
c) mixing the at least one gelling agent with the at least one ionic-modifying agent, thereby triggering, promoting and/or modulating interactions within the biogel composition leading to the formation and/or stabilization of a three-dimensional polymeric network having viscoelastic behaviour; the viscoelastic behaviour resulting, at least in part, from interactions within the biogel composition.

The method for producing a biogel composition according to the present disclosure may further comprise:
- the addition of at least one hydration-modulating agent simultaneously with or after step a);
- the addition of at least one thickening agent simultaneously with step a) and/or after step b);
- the addition of at least one crosslinking agent simultaneously with or after step b);
- the addition of at least one antioxidant agent simultaneously with and/or after step b);
and may be carried out under sterile or aseptic conditions.

### DESCRIPTION OF THE FIGURES

The following figure provides preferred embodiments of the present disclosure and should not be understood as limiting its scope.

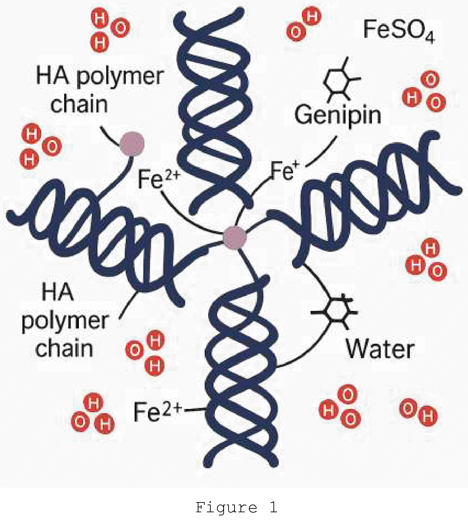

Figure 1 represents an embodiment of a biogel composition for multitissue regeneration (1) according to the present disclosure, comprising a gelling agent (10), an ionic-modifying agent (20) comprising ferrous ions (Fe²⁺) (21), optionally originating from an iron salt (23), in particular ferrous sulfate (FeSO₄) (24), and a hydration-modulating agent (30), in particular hyaluronic acid (32). The biogel composition (1) may further comprise a crosslinking agent (40), in particular genipin. Interactions (101) between the gelling agent (10) and the ionic-modifying agent (20), optionally in combination with the crosslinking agent (40), may contribute to the formation and/or stabilization of a three-dimensional polymeric network (100), the hydration-modulating agent (30) contributing to water retention within the biogel composition (1) and to the modulation of at least one rheological property (106), the said three-dimensional polymeric network (100) having viscoelastic behavior (107), and the crosslinking agent (40) further contributing to reinforcing the three-dimensional polymeric network (100) and enhancing its structural stability.

### DETAILED DESCRIPTION OF THE DISCLOSURE

The more general configurations of the present disclosure are presented in the section "SUMMARY OF THE DISCLOSURE". These are further detailed below according to preferred and/or advantageous embodiments of the present disclosure.

Within the scope of the present disclosure, the expression "biogel composition" (1) refers to an aqueous formulation that may be in a liquid, semisolid, or gel-forming state, and is capable of forming a three-dimensional polymeric network (100) exhibiting viscoelastic behavior (107), suitable for application onto or within at least one biological surface (2). The expression "three-dimensional polymeric network" (100) refers to a cohesive structure resulting from physical and/or chemical interactions (101) within and/or between polymer chains, including ionic (51), covalent and/or intermolecular interactions (102, 103, 104), capable of retaining water (40) and imparting modulable rheological properties (106), including viscoelastic behavior (107), to the biogel composition (1). The expression "biological surface" (2) refers to any living tissue, anatomical structure, or biological interface, including external or internal surfaces of organs, soft or hard tissues, body cavities, and interfaces resulting from surgical, traumatic, or pathological conditions, and comprises superficial, intermediate, or deep regions such as skin, mucosae, cornea, connective tissue, bone, cartilage, and/or joints.

In a preferred embodiment, the biogel composition (1) of the present disclosure comprises:
- at least one gelling agent (10) configured to form or contribute to the formation of a three-dimensional polymeric network (100), thereby providing structural integrity to the biogel composition (1); and
- at least one ionic-modifying agent (20) comprising ferrous ions (Fe²⁺) (21) and/or ferric ions (Fe³⁺) (22), in a concentration sufficient to trigger and/or modulate interactions (101) within the biogel composition (1), thereby promoting the formation and/or stabilization of the three-dimensional polymeric network (100);
the three-dimensional polymeric network (100) having viscoelastic behavior (107) resulting, at least in part, from the said interactions (101) within the biogel composition (1), enabling adaptation of the biogel composition (1) to the morphology of at least one biological surface (2).

The interactions (101) triggered and/or modulated by the at least one ionic-modifying agent (20) within the biogel composition (1) may comprise at least ionic interactions (102) with the at least one gelling agent (10), thereby forming ionic crosslinking points (105). These ionic crosslinking points (105) contribute to the formation and stabilization of the three-dimensional polymeric network (100), which may present at least one modulable rheological property (106), including viscoelastic behavior (107).

The at least one rheological property (106) of the biogel composition (1) may relate to at least one mechanical parameter characterizing its response to deformation induced by an external force, including compression, shear, or tension, for example during administration. Such at least one rheological property (106) may include viscosity, elasticity, hardness, viscoelasticity, thixotropy, plasticity, shear-thinning behavior, post-shear structural recovery, and/or the dynamic storage (G') and loss (G") moduli.

The at least one rheological property (106) may be modulable as a function of parameters including pH, ionic concentration, and/or the redox state of the biogel composition (1), as well as the conditions of the biological surface (2) to which it is applied - for instance, tissue type, temperature, and/or similar physiological factors. In this context, the term "redox state" refers to the ratio between ferrous ions (Fe²⁺) (21) and ferric ions (Fe³⁺) (22) within the biogel composition (1). The modulation of at least one rheological property (106) enables adjustment of the mechanical behavior of the biogel composition (1) to the morphology of at least one biological surface (2) and/or to clinical requirements of a therapeutic intervention, including the mode of administration and the intended use or application, such as filling (500), coating (501), sealing (502), protective (503), stabilization (504), and/or regeneration (505) material for at least one biological surface (2).

The at least one ionic-modifying agent (20) may comprise at least one iron salt (23) selected from ferrous sulfate (FeSO₄) (24), ferric sulfate (Fe₂(SO₄)₃) (25), or any combination thereof, preferably ferric sulfate (Fe₂(SO₄)₃) (25). The use of sulfate salts (24, 25) provides high aqueous solubility and efficient ionic dissociation, thereby ensuring the availability of ferrous ions (Fe²⁺) (21) and/or ferric ions (Fe³⁺) (22) for interaction (101) within the biogel composition (1). This configuration of the at least one ionic-modifying agent (20) also contributes to controlling the density of interactions (101) within the three-dimensional polymeric network (100), enhancing its structural stability and enabling modulation of its rheological properties (106). The at least one ionic-modifying agent (20) further provides hemostatic properties to the biogel composition (1), promoting coagulation, reducing bleeding, and supporting biological processes associated with wound healing and tissue regeneration, on or within at least one biological surface (2).

The biogel composition (1) of the present disclosure may further comprise at least one hydration-modulating agent (30) configured to bind and/or retain water and to enhance the hydration capacity of the biogel composition (1), thereby contributing to its rheological properties (106). Such hydration-modulating agent (30) enables control of the water content of the biogel composition (1) and improves its biocompatibility and mechanical adaptability, thereby optimizing its performance across different clinical applications, including dermatology, orthopedics, plastic surgery, dentistry, and/or ophthalmology.

In a particular embodiment, the at least one hydration-modulating agent (30) may comprise at least one polysaccharide (31), synthetic polymer, protein, or any combination thereof, preferably a polysaccharide (31) capable of interacting with water and contributing to the formation and/or stabilization of the three-dimensional polymeric network (100). Such polysaccharide (31) enhances the water-binding capacity of the biogel composition (1) and promotes the formation of a structured and hydrated three-dimensional polymeric network (100), due to its inherent ability to interact with water and form a polymeric matrix capable of supporting interactions (101), such as intermolecular interactions (104), thereby contributing to the performance of the biogel composition (1).

The polysaccharide (31) may, preferably, be hyaluronic acid (32), which may have a molecular weight between 300000 Da and 2000000 Da, preferably between 500000 Da and 2000000 Da. The inclusion of hyaluronic acid (32) enhances the biocompatibility of the biogel composition (1) due to its structural and functional similarity to endogenous hyaluronic acid (32), and promotes the formation of a hydrated and viscoelastic three-dimensional polymeric network (100), thereby contributing to the lubrication of the at least one biological surface (2) and to the modulation of at least one rheological property (106) of the biogel composition (1).

In an alternative configuration, the hyaluronic acid (32) may be of marine origin, namely derived from algae of the genera *Gracilaria spp.* or *Porphyra spp.* Such configuration provides a biocompatible and biodegradable source of hyaluronic acid (32) while maintaining its functional properties within the biogel composition (1), and may further contribute to the sustainability of the product and/or the production process.

The biogel composition (1) of the present disclosure may further comprise at least one crosslinking agent (40) configured to reinforce and stabilize the three-dimensional polymeric network (100), preferably by increasing the number of intermolecular interactions (104) within and/or between polymer chains of the at least one gelling agent (10). The at least one crosslinking agent (40) may be selected from genipin, BDDE, carbodiimides, tannic acid, or any combination thereof; wherein genipin may act as a natural crosslinking agent (40), and tannic acid may additionally confer bioactive properties to the biogel composition (1), including antioxidant and/or antimicrobial activity. The selection of these crosslinking agents (40) enables controlled formation of intermolecular interactions (104) under controlled pH and temperature conditions compatible with at least one biological surface (2), thereby allowing modulation of the structural properties of the three-dimensional polymeric network (100), including crosslinking density and three-dimensional polymeric network (100) stability. Within the context of the present disclosure, the term "crosslinking density" refers to the number and/or distribution of interactions (101) within and/or between polymer chains forming the three-dimensional polymeric network (100), which influences the compactness and cohesion of the said three-dimensional polymeric network (100). The term "three-dimensional polymeric network stability" (100) refers to the ability of the three-dimensional polymeric network (100) to maintain its structure and functionality over time when subjected to a physiological moist environment, mechanical stress, and/or degradation processes. The physiological moist environment may comprise the presence of at least one biological fluid, in particular blood, saliva, interstitial fluid, and/or exudate.

The biogel composition (1) of the present disclosure may further comprise at least one thickening agent (50) configured to modulate the rheological properties (106) and/or to stabilize the biogel composition (1), thereby promoting its retention on at least one biological surface (2), including under a physiological moist environment. The at least one thickening agent (50) may be selected from carboxymethylcellulose, carbomer, polyvinylpyrrolidone, poloxamers, hydroxyethyl cellulose, hydroxypropyl cellulose, gelatin, agarose, or any combination thereof, and may further include polycarbophil, polyvinyl alcohol, Methyl Vinyl Ether-Maleic Anhydride (PMV/AM) copolymer, and/or Polyvinylpyrrolidone K30 (PVP K30). The presence of the at least one thickening agent (50), in combination with the at least one gelling agent (10), enables modulation of at least one rheological property (106) of the biogel composition (1), including viscosity and shear-thinning behavior, thereby improving its handling during administration and promoting its retention on or within at least one biological surface (2), without substantially altering the formation of the three-dimensional polymeric network (100). This configuration further improves the resistance of the biogel composition (1) to displacement under a physiological moist environment, thereby contributing to a barrier effect on or within the at least one biological surface (2) and limiting exposure to external contaminating and/or infectious agents.

In an advantageous embodiment, the biogel composition (1) may further comprise at least one antioxidant agent (60) configured to limit the oxidation of the at least one ionic-modifying agent (20). This at least one antioxidant agent (60) may be selected from ascorbic acid, sodium ascorbate, glutathione, N-acetylcysteine, and/or any combination thereof; preferably ascorbic acid, which acts as a reducing agent capable of maintaining the availability of ferrous ions (Fe²⁺) (21) within the biogel composition (1). The presence of the at least one antioxidant agent (60) contributes to controlling the redox state of the biogel composition (1), thereby supporting the formation and stability of the three-dimensional polymeric network (100). In addition, the at least one antioxidant agent (60), in particular the ascorbic acid, contributes to protecting the biological surface (2) against reactive oxygen species and to supporting healing and/or regeneration processes.

The biogel composition (1) of the present disclosure may be configured to provide controlled release (200) of at least one bioactive agent (70), selected from a pharmacological agent, an antimicrobial agent (71), an anti-inflammatory agent, a tissue regeneration-promoting agent, a protein agent, a peptide agent, a biodegradable nanostructured agent, a liposomal system, and/or any combination thereof. Within the context of the present disclosure, the expression "controlled release" (200) refers to the gradual diffusion of the at least one bioactive agent (70) through the three-dimensional polymeric network (100) and/or to its release through controlled degradation of the said three-dimensional polymeric network (100). This controlled release (200) of at least one bioactive agent (70) enables the maintenance of predefined concentrations, namely therapeutic concentrations, over time on or within at least one biological surface (2), thereby reducing, for example, the need for repeated administrations of the biogel composition (1) and/or of the bioactive agent (70).

In particular, when the at least one bioactive agent (70) comprises an antimicrobial agent (71), the controlled release (200) may confer antimicrobial activity to the biogel composition (1), including antibacterial and/or antifungal activity. Within the context of the present disclosure, the expression "antimicrobial activity" refers to the ability to inhibit the growth and/or inactivate microorganisms, including bacteria and fungi, through mechanisms comprising disruption of the cell wall and/or membrane and/or the generation of reactive oxygen species, thereby creating a microenvironment unfavorable to microbial proliferation, limiting contamination of the biological surface (2) and supporting healing and/or regeneration thereof.

The biogel composition (1) of the present disclosure may optionally comprise at least one buffer system (80) configured to maintain the pH of the biogel composition (1) within a physiologically compatible range (81), preferably between 4.0 and 7.5, more preferably between 5.0 and 6.5. The at least one buffer system (80) may be selected from phosphates, citrates, acetates, bicarbonates, TRIS, or any combination thereof, preferably phosphates and/or citrates.

Maintaining the pH within this physiologically compatible range (81) supports the stability of the components of the biogel composition (1) and enables controlled formation of the three-dimensional polymeric network (100), in particular by maintaining ferrous ions (Fe²⁺) (21) and/or ferric ions (Fe³⁺) (22) in a form available for interaction within the biogel composition (1). The physiologically compatible range (81) further allows modulation of the structure of the three-dimensional polymeric network (100) and the corresponding at least one rheological property (106), thereby favoring retention of the biogel composition (1) on or within at least one biological surface (2).

The biogel composition (1) of the present disclosure may be provided as a mucoadhesive gel (300), foam (301), or other liquid or semisolid form (302, 303), and may be configured to be administered by, for instance, direct application, injection, spraying, or three-dimensional deposition. After administration, the biogel composition (1) may be configured to form at least one continuous adherent layer (304) on at least one biological surface (2), thereby enabling conformity to the morphology thereof and retention thereon. This configuration further enables sealing of the at least one biological surface (2) by forming a stable interface that provides a microenvironment favorable to healing and/or regeneration of the at least one biological surface (2), while limiting exposure to contaminating and/or infectious agents. In the context of the present disclosure, "mucoadhesive gel" (300) refers to a hydrated semisolid matrix configured to be applied onto or within a biological surface (2), in particular a mucosa, and capable of adhering thereto, thereby promoting retention of the biogel composition (1) on or within the at least one biological surface (2). The expression "continuous adherent layer" (304) refers to a film or coating formed by the biogel composition (1) after its administration, acting as a protective interface on or within the at least one biological surface (2), irrespective of its thickness.

The biogel composition (1) of the present disclosure may further be configured to present, on or within at least one biological surface (2), a controlled absorption time (201) greater than that of the at least one gelling agent (10) in the absence of the at least one ionic-modifying agent (20), optionally under a physiological moist environment. This increased controlled absorption time (201) may result, in particular, from stabilization of the three-dimensional polymeric network (100) by interactions (101) within the biogel composition (1), which enhance its resistance to degradation, including enzymatic degradation, thereby contributing to prolonged retention of the biogel composition (1) on or within at least one biological surface (2) and reducing the need for repeated administration.

The present disclosure further relates to a system for administering the biogel composition (1, 400), comprising at least one biogel composition (1) as described herein and at least one device (410) configured to store, prepare, handle and/or administer the biogel composition (1) onto or within at least one biological surface (2). The at least one device (410) may comprise, alone or in combination:
- at least one storage device (411) configured to receive and contain the biogel composition (1), such as a container, cartridge, tube, or equivalent means;
- at least one mixing and/or activating device (412) configured to combine and/or homogenize components of the biogel composition (1), including at least one gelling agent (10), at least one ionic-modifying agent (20), and/or at least one additional component, such as a multi-chamber system, an auto-mixing tip, or equivalent means; and/or
- at least one administration device (413) configured to apply the biogel composition (1), directly or indirectly, onto or within at least one biological surface (2), such as a syringe, cannula, or equivalent means. This system for administering the biogel composition (1, 400) enables storage, preparation, and controlled administration of the biogel composition (1) onto or within at least one biological surface (2), thereby reducing administration variability and enabling formation of a three-dimensional polymeric network (100) having viscoelastic behavior (107) and suitable structural stability. The referred system (400) further enables adaptation of the administration of the biogel composition (1) to the morphology of at least one biological surface (2) and to the clinical requirements of a therapeutic intervention.

The biogel composition (1) of the present disclosure may be applied in a variety of contexts and on or within at least one biological surface (2) comprising different types of cells and/or tissues. This versatility enables its use as a filling (500), coating (501), sealing (502), protective (503), stabilizing (504) and/or regeneration (505) material across multiple clinical fields, including dermatology, orthopedics, plastic surgery, dentistry, and/or ophthalmology.

For example, the biogel composition (1) of the present disclosure may be used as a substitute for or complement to at least one filling material (501), such as collagen membranes, bone substitutes, connective-tissue substitutes, and/or autologous grafts, providing conformable filling of defects in at least one biological surface (2), temporary structural support, and a microenvironment favorable to cell adhesion and to healing and/or regeneration thereof. Owing to its controlled absorption time (201), which may be greater than that of the at least one gelling agent (10) in the absence of the at least one ionic-modifying agent (20), the biogel composition (1) remains effective as a filling material (500) for a desired period and is subsequently absorbed in a controlled manner, without requiring removal, thereby reducing the presence of residual material on or within at least one biological surface (2), while supporting healing and/or regeneration processes.

In an advantageous embodiment, the biogel composition (1) as described herein may also be used as a substitute for or complement to at least one suture material, promoting stabilization of at least one biological surface (2) through the formation of a three-dimensional polymeric network (100) having viscoelastic behavior (107), while being absorbable and not requiring removal, thereby reducing the risk of infection and supporting healing and/or regeneration processes.

The biogel composition (1) of the present disclosure may also be used as a hemostatic agent and/or as a promoter of healing and/or regeneration of at least one biological surface (2), enabling rapid control of bleeding and induction of local hemostasis, while forming a protective interface that limits exposure of the biological surface (2) to contaminating and/or infectious agents. These effects may result, at least in part, from the structure of the three-dimensional polymeric network (100) and at least one rheological property (106) of the biogel composition (1).

Additionally, the biogel composition (1) as described herein may be used in three-dimensional bioprinting via digital modelling (406), preferably through CAD and/or CAM, enabling precise conformation to the morphology of at least one biological surface (2). For this application, at least one rheological property (106) of the biogel composition (1) may be modulated to enable controlled deposition, maintain printing resolution, and ensure structural stability after administration, thereby enabling the formation of a three-dimensional polymeric network (100) adapted to the morphology of the at least one biological surface (2) and/or to the clinical requirements of a therapeutic intervention.

The present disclosure further relates to a method for producing a biogel composition (1, 600) according to the present disclosure, comprising the steps of:
a) providing at least one gelling agent (10), preferably in an aqueous phase (610), thereby enabling dispersion of the at least one gelling agent (10) and contributing to the formation of a homogeneous medium suitable for subsequent interactions (101) within the biogel composition (1);
b) providing at least one ionic-modifying agent (20) comprising ferrous ions (Fe²⁺) (21) and/or ferric ions (Fe³⁺) (620), thereby enabling interactions (101) within the biogel composition (1) and contributing to the redox state of the biogel composition (1);
c) mixing the at least one gelling agent (10) with the at least one ionic-modifying agent (20, 630), thereby triggering and/or promoting interactions (101) within the biogel composition (1) leading to the controlled formation and/or stabilization of a three-dimensional polymeric network (100) having viscoelastic behaviour (107); the viscoelastic behaviour (107) resulting, at least in part, from interactions (101) within the biogel composition (1).

The sequential execution of the steps (610, 620, 630) enables controlled formation of the three-dimensional polymeric network (100), thereby contributing to the stability and reproducibility of the biogel composition (1), and enabling modulation of its at least one rheological property (106).

The method for producing a biogel composition (1, 600) as described herein may further comprise at least one of the following steps:
- the addition of at least one hydration-modulating agent (30) simultaneously with or after step a) (640), configured to bind and/or retain water and to contribute to at least one rheological property (106) of the biogel composition (1);
- the addition of at least one thickening agent (50) simultaneously with step a) and/or after step b) (650), configured to contribute to the modulation and/or stabilization of the biogel composition (1), including under a physiological moist environment, thereby promoting retention on or within at least one biological surface (2);
- the addition of at least one crosslinking agent (40) simultaneously with or after step b) (660), configured to contribute to reinforcement of the three-dimensional polymeric network (100), thereby enhancing its cohesion and structural stability;
- the addition of at least one antioxidant agent (60) simultaneously with and/or after step b) (670), configured to limit oxidation of ferrous ions (Fe²⁺) (21) and to contribute to the redox state of the biogel composition (1), thereby supporting stability of the three-dimensional polymeric network (100).

These steps (610, 620, 630, 640, 650, 660, 670) of the method for producing a biogel composition (1, 300) as described herein may be performed under sterile or aseptic conditions, thereby preserving the structural integrity and stability of the biogel composition (1) and limiting contamination by contaminating and/or infectious agents.

The present disclosure further relates to a method for administering a biogel composition (1, 700) onto or within at least one biological surface (2), using a system for administering a biogel composition (1, 400) as described herein. According to this method (700), the three-dimensional polymeric network (100) may be formed either (i) *in situ*, following administration of the biogel composition (1), or (ii) *ex situ*, prior to its administration. In the *in situ* configuration, the biogel composition (1) may be applied onto or within at least one biological surface (2), wherein the formation of the three-dimensional polymeric network (100) may occur following administration, as a result of interactions (101) between at least one gelling agent (10) and at least one ionic-modifying agent (20). These interactions (101) may be initiated upon contact and/or mixing of the said components of the biogel composition (1) on or within the at least one biological surface (2), thereby leading to the formation and/or stabilization of the three-dimensional polymeric network (100) directly at the at least one biological surface (2). At least one additional component of the biogel composition (1), as described herein, may be added on or within at least one biological surface (2), thereby contributing to the stabilization and/or reinforcement of the three-dimensional polymeric network (100), the modulation of at least one rheological property (106) of the biogel composition (1), and/or the control of at least one physicochemical and/or biological property of the biogel composition (1), as described herein. This *in situ* formation of the three-dimensional polymeric network (100) enables localized formation thereof, thereby facilitating conformity to the morphology of at least one biological surface (2). Alternatively, in the *ex situ* configuration, the three-dimensional polymeric network (100) may be formed prior to administration, for example by mixing and/or allowing interaction (101) between at least one gelling agent (10) and at least one ionic-modifying agent (20) outside the at least one biological surface (2), thereby providing a biogel composition (1) having at least one predefined rheological property (106), such as viscoelastic behavior (107). This configuration enables controlled formation of the three-dimensional polymeric network (100), thereby facilitating adjustment of the said at least one rheological property (106) according to the administration conditions, including the nature of at least one biological surface (2) and the clinical requirements of the therapeutic intervention.

As will be evident to a person skilled in the art, the present disclosure should not be limited to the embodiments described herein, and various modifications remain possible within the scope of the present disclosure.

Evidently, the preferred embodiments presented above are combinable in the different possible forms, being the repetition of all such combinations avoided herein.

## Claims

**1.** A biogel composition for multitissue regeneration (1), **characterized in that** it comprises:
- at least one gelling agent (10) configured to form or contribute to the formation of a three-dimensional polymeric network (100);
- at least one ionic-modifying agent (20) comprising ferrous ions (Fe²⁺) (21) and/or ferric ions (Fe³⁺) (22), configured to trigger, promote and/or modulate interactions (101) within the biogel composition (1), thereby leading to the formation and/or stabilization of the said three-dimensional polymeric network (100);
the three-dimensional polymeric network (100) having viscoelastic behavior (107), resulting, at least in part, from interactions (101) within the biogel composition (1).

**2.** The biogel composition (1) according to the previous claim, **characterized in that** the interactions (101) triggered, promoted and/or modulated by the at least one ionic-modifying agent (20) within the biogel composition (1) comprise at least ionic interactions (102) with the at least one gelling agent (10).

**3.** The biogel composition (1) according to any of the preceding claims, **characterized in that** the at least one ionic-modifying agent (20) comprises at least one iron salt (23) selected from ferrous sulfate (FeSO₄) (24), ferric sulfate (Fe₂(SO₄)₃) (25), or any combination thereof.

**4.** The biogel composition (1) according to any of the preceding claims, **characterized in that** it further comprises at least one hydration-modulating agent (30) configured to bind and/or retain water and to contribute to at least one rheological property (106) of the biogel composition (1); the at least one rheological property (106) comprising at least one mechanical parameter selected from viscosity, elasticity, plasticity, shear-thinning behavior, and/or post-shear structural recovery, preferably **characterized in that** the at least one hydration-modulating agent (30) comprises at least one polysaccharide (31), synthetic polymer, protein, or combination thereof; preferably a polysaccharide (31), even more preferably wherein the polysaccharide (31) is hyaluronic acid (32); the hyaluronic acid (32) having a molecular weight between 300000 Da and 2000000 Da, preferably between 500000 Da and 2000000 Da.

**4.** The biogel composition (1) according to any of the preceding claims, **characterized in that** it further comprises at least one crosslinking agent (40) configured to reinforce and stabilize the three-dimensional polymeric network (100), preferably wherein the at least one crosslinking agent (40) is selected from genipin, 1,4-Butanediol Diglycidyl Ether (BDDE), carbodiimides, tannic acid, or any combination thereof.

**5.** The biogel composition (1) according to any of the preceding claims, **characterized in that** it further comprises at least one thickening agent (50) configured to contribute to the modulation and/or stabilization of the biogel composition (1), including in a physiological moist environment, preferably wherein the at least one thickening agent (50) is selected from carboxymethylcellulose, carbomer, polyvinylpyrrolidone, poloxamers, hydroxyethyl cellulose, hydroxypropyl cellulose, gelatin, agarose, or any combination thereof, optionally wherein the physiological moist environment comprises the presence of at least one biological fluid selected from blood, saliva, interstitial fluid, exudate, or any combination thereof.

**6.** The biogel composition (1) according to any of the preceding claims, **characterized in that** it further comprises at least one antioxidant agent (60) configured to limit the oxidation of the at least one ionic-modifying agent (20), preferably wherein the at least one antioxidant agent (60) is selected from ascorbic acid, sodium ascorbate, glutathione, N-acetylcysteine, or any combination thereof.

**7.** The biogel composition (1) according to any of the preceding claims, **characterized in that** it is configured to provide controlled release (200) of at least one bioactive agent (70); the controlled release (200) of the at least one bioactive agent (70) comprising the gradual diffusion of the said bioactive agent (70) through the three-dimensional polymeric network (100) and/or through controlled degradation of that three-dimensional polymeric network (100), preferably wherein the at least one bioactive agent (70) is selected from an antimicrobial agent (71), an anti-inflammatory agent, a tissue-regeneration-promoting agent; a protein; a peptide; a nanostructured agent; a liposomal system; and/or any combination thereof, even more preferably wherein the controlled release (200) of at least one antimicrobial agent (71) is configured to confer antimicrobial activity, including antibacterial and/or antifungal activity, to the biogel composition (1).

**8.** The biogel composition (1) according to any of the preceding claims, **characterized in that** it is configured as a mucoadhesive gel (300), foam (301), or in a liquid or semisolid form (302, 303), and/or configured to form, upon administration onto or within at least one biological surface (2), at least one continuous adherent layer (304).

**9.** The biogel composition (1) according to any of the preceding claims, **characterized in that** it is configured to present, on or within at least one biological surface (2), a controlled absorption time (201) greater than that of the at least one gelling agent (10) in the absence of the at least one ionic-modifying agent (20).

**10.** A system for administering a biogel composition (1, 400) according to any of the preceding claims 1-9, **characterized in that** it comprises at least one biogel composition (1) and at least one device configured to prepare, handle, and/or administer the biogel composition (1, 220).

**11.** Biogel composition (1) according to any of the preceding claims 1-9 for use as a filling (500), coating (501), sealing (502), protective (503), stabilizing (504) and/or regeneration material (505) of at least one biological surface (2) or as a substitute for or complement to at least one filling material (500) selected from collagen membranes, bone substitutes, connective-tissue substitutes, autologous grafts, and/or at least one suture material or as a hemostatic agent and/or for promoting healing and/or regeneration of at least one biological surface (2) or in three-dimensional bioprinting via digital modelling (406), preferably through Computer-Aided Design (CAD) and/or Computer-Aided Manufacturing (CAM).

**12.** A method for producing a biogel composition (1, 600) according to any of the preceding claims 1-9, **characterized in that** it comprises the steps of:
a) providing at least one gelling agent (10), preferably in an aqueous phase (610);
b) providing at least one ionic-modifying agent (20) comprising ferrous ions (Fe²⁺) (21) and/or ferric ions (Fe³⁺) (22, 620);
c) mixing the at least one gelling agent (10) with the at least one ionic-modifying agent (20, 630), thereby triggering, promoting and/or modulating interactions (101) within the biogel composition (1) leading to the formation and/or stabilization of a three-dimensional polymeric network (100) having viscoelastic behaviour (107); the viscoelastic behaviour (107) resulting, at least in part, from interactions (101) within the biogel composition (1);
d) optionally further comprising the addition of at least one hydration-modulating agent (30) simultaneously with or after step a) (640);
e) optionally further comprising the addition of at least one thickening agent (50) simultaneously with step a) and/or after step b) (650).

**13.** The method for producing a biogel composition (1, 300) according to claim 12, **characterized in that** it further comprises the addition of at least one crosslinking agent (40) simultaneously with or after step b) (660).

**14.** The method for producing a biogel composition (1, 300) according to claim 12, **characterized in that** it comprises the addition of at least one antioxidant agent (60) simultaneously with and/or after step b) (670).

**15.** The method for producing a biogel composition (1, 300) according to claim 12, **characterized in that** it is performed under sterile or aseptic conditions.
